# EUROPEAN PATENT SPECIFICATION

(11) **EP 4 356 133 B1**
(45) Date of publication and mention of the grant of the patent: **30.07.2025**
(21) Application number: 22734277.1
(22) Date of filing: 15.06.2022
(51) Int. Cl.: G01N 33/543, C12Q 1/6804, B82Y 15/00, B82Y 30/00, B82Y 25/00, H01F 10/32

(54) **METHOD FOR IMMUNOSENSING ON A LIPID LAYER USING MAGNETIC TUNNEL JUNCTIONS II**
VERFAHREN ZUR IMMUNMESSUNG AUF EINER LIPIDSCHICHT MITTELS MAGNETISCHER TUNNELÜBERGÄNGE II
PROCÉDÉ D'IMMUNODÉTECTION SUR UNE COUCHE DE LIPIDES UTILISANT DES JONCTIONS À EFFET TUNNEL MAGNÉTIQUE II

(30) Priority: 17.06.2021 EP 21179975
(43) Date of publication of application: 24.04.2024
(73) Proprietor: Roche Diagnostics GmbH, 68305 Mannheim (DE); F. Hoffmann-La Roche AG, 4070 Basel (CH)
(72) Inventor: BARANOWSKI, Regina, 82377 Penzberg (DE); HEINDL, Dieter, 82377 Penzberg (DE); STENGELE, Nikolaus-Peter, 82377 Penzberg (DE); WELLNER, Christian, 82377 Penzberg (DE)
(74) Representative: Altmann Stößel Dick Patentanwälte PartG mbB
(86) International application number: PCT/EP2022/066364
(87) International publication number: WO 2022/263542

(56) References cited:
- WO-A1-2015/175856
- WO-A1-2016/161402
- WO-A1-2018/067878
- WO-A1-2020/023503
- WO-A1-2020/077309
- WO-A1-2020/180645
- WO-A2-2007/024676
- WO-A2-2007/092909
- US-A1- 2007 154 881
- US-A1- 2014 272 939

## Description

The present invention concerns the field of diagnostics. In particular, it relates to a method for determining an analyte suspected to be present in a sample comprising contacting said sample with a sensor element comprising an anchor layer which is present on a solid support, a first binding agent which is capable of specifically binding to the analyte and which is anchored in the anchor layer, a second binding agent which is capable of specifically binding to the analyte when bound to the first binding agent and which is immobilized on the solid support, said second binding agent comprising at least one magnetic label, and a magnetic tunnel junction in functional proximity to the second binding agent which generates a signal elicited in proximity to the at least one magnetic label of the second binding agent, for a time and under conditions which allow for specific binding of the analyte suspected to be present in the sample to the first binding agent and specific binding of the second binding agent to the analyte bound to the first binding agent and detecting the formation the complex of first binding agent, analyte and second binding agent based on an altered signal which is generated by the magnetic tunnel junction whereby the analyte is determined. The present invention, further, relates to a device and a kit for determining an analyte suspected to be present in a sample and to the use of said device for determining an analyte suspected to be present in a sample.

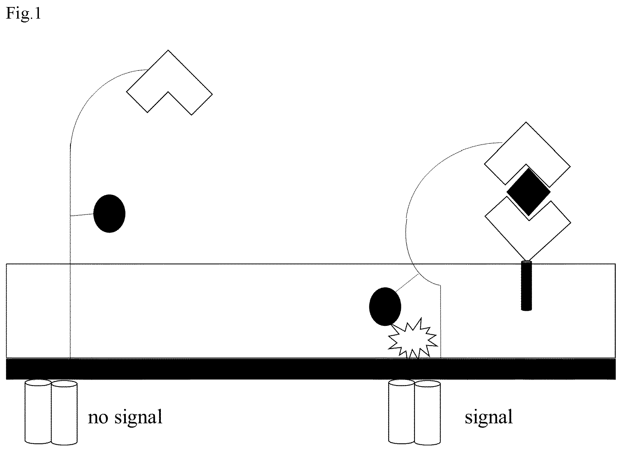

WO 2015/175856, as well as WO 2020/180645 and US 2014/272939 disclose a method comprising the formation of a complex of the analyte of interest, a capture reagent present on a surface that comprises an anchoring region, and a detection reagent linked to a nucleic acid probe. The principle underlying the detection in the method of these documents is based on an extension process resulting in the extended sequence that forms the anchoring region; see e.g. Fig. 1(a) and page 3, lines 22-24 of WO 2015/175856 describing: "extending the probe to form an extended sequence comprising an anchoring region that binds the anchoring reagent: binding the extended sequence to the anchoring reagent: and measuring the amount of extended sequence bound to the surface." Thus, the anchoring region of these documents is formed by "extending the probe to form an extended sequence comprising an anchoring region that binds the anchoring reagent". Hence, the anchoring region of these documents is designed to engage in binding to the anchoring reagent of the capture reagent. WO 2007/024676 relates to methods for detecting at least one target analyte in a sample. The methods comprise using a target analyte with at least two binding sites; the target analyte may be bound by two different types of capture probes and optionally a type of detector probe (see page 4, lines 10-18). A first capture probe is provided on a substrate, i.e. could be interpreted as being immobilized on a support. However, the further probes do not appear to be anchored in an anchor layer. WO 2007/024676 discloses a coated surface as a suitable substrate (page 8, line 21 to page 9, line 4). This coat on a substrate might be interpreted as an anchor layer on a solid support.

Immunoassays are widely used for various diagnostic purposes. Several setups have been developed for immunoassays. One of the most popular immunoassays is the enzyme-linked sorbent immunoassay (ELISA).

In ELISA, a liquid sample containing an analyte of interest or suspected to contain an analyte of interest is applied onto a stationary solid phase with special binding properties due to the presence of antibodies or antibody-like molecules such as aptamers. After sample application, multiple reagents are sequentially added, incubated, and washed away in order to carry out and stop an anytical detection reaction. After carrying out all these steps, physical or chemical properties in the setup, usually the liquid phase, are changed that can be detected. Typically, optical change such as color development by the product of an enzymatic reaction will happen in the final liquid phase. These changes correlate to the presence or abundance of the analyte of interest present in the investigated sample. The typically quantitative read-out is usually based on detection of intensity of transmitted light by spectrophotometry, which involves quantitation of transmission of some specific wavelength of light through the liquid. The sensitivity of detection depends on amplification of the signal during the analytic reactions. Since enzyme reactions are very well known amplification processes, the signal is generated by enzymes which are linked to the detection reagents in fixed proportions to allow accurate quantification.

For an ELISA setup, the analyte binding agent, e.g., an antibody, is immobilized on a solid phase such as a solid support structure. Usually, the antibody is coated and dried onto the transparent bottom and sometimes also side wall of a well in an analytic multi-well plate or in an analytic vial. However, nanoparticles or other beads can also be used as solid phases for ELISAs.

For research and diagnostic, ELISAs are often used in the format of so-called sandwich ELISAs. In a sandwich format, an immobilized capture antibody is used to capture, i.e. specifically bind to, an analyte present in a sample applied to said immobilized antibody. After the capture antibody has specifically bound to the analyte, the sample material is washed away. In a subsequent step the analyte bound to the immobilized antibody is incubated with a detection antibody that specifically binds to the analyte or the complex of analyte and capture antibody. The detection antibody typically contains a detectable linker or an adaptor molecule which allows for attracting such detectable labels from a solution.

However, in light of the fragile immune complexes which are required for signal generation and the various components used for such an ELISA in the sandwich format, there are many possibilities for undesired binding of detection antibodies and thus for the generation of false positive signals. Therefore, sandwich ELISAs used for research often need validation because of the risk of false positive results. Moreover, there are limitations with respect to sensitivity and specificity due to the various drawbacks of such a fragile multi-component assay format.

The technical problem underlying the present invention may be seen as the provision of means and methods for complying with the aforementioned needs. The technical problem is solved by the embodiments characterized in the claims and herein below.

The present invention relates to a method for determining an analyte suspected to be present in a sample comprising:
(a) contacting said sample with a sensor element comprising:
   (i) an anchor layer which is present on a solid support;
   (ii) a first binding agent which is capable of specifically binding to the analyte and which is anchored in the anchor layer;
   (iii) a second binding agent which is capable of specifically binding to the analyte when bound to the first binding agent and which is immobilized on the solid support, said second binding agent comprising at least one magnetic label; and
   (iv) a magnetic tunnel junction in functional proximity to the second binding agent which generates a signal elicited in proximity to the at least one magnetic label of the second binding agent;
   for a time and under conditions which allow for specific binding of the analyte suspected to be present in the sample to the first binding agent and specific binding of the second binding agent to the analyte bound to the first binding agent; and
(b) detecting the formation the complex of first binding agent, analyte and second binding agent based on an altered signal which is generated by the magnetic tunnel junction whereby the analyte is determined.

It is to be understood that in the specification and in the claims, "a" or "an" can mean one or more, depending upon the context in which it is used. Thus, for example, reference to "an" item can mean that at least one item can be utilized.

As used in the following, the terms "have", "comprise" or "include" are meant to have a non-limiting meaning or a limiting meaning. Thus, having a limiting meaning these terms may refer to a situation in which, besides the feature introduced by these terms, no other features are present in an embodiment described, i.e. the terms have a limiting meaning in the sense of "consisting of" or "essentially consisting of". Having a non-limiting meaning, the terms refer to a situation where besides the feature introduced by these terms, one or more other features are present in an embodiment described.

Further, as used in the following, the terms "preferably", "more preferably", "most preferably", "particularly", "more particularly", "typically", and "more typically" or similar terms are used in conjunction with additional or alternative features, without restricting alternative possibilities.

Further, it will be understood that the term "at least one" as used herein means that one or more of the items referred to following the term may be used in accordance with the invention. For example, if the term indicates that at least one item shall be used this may be understood as one item or more than one item, i.e. two, three, four, five or any other number. Depending on the item the term refers to the skilled person understands as to what upper limit the term may refer, if any.

The method according to the present invention may consist of the aforementioned steps (a) and (b) or may comprise further steps, such as pretreating or isolating the sample prior to step (a) and/or after step (b) further one or more steps of evaluating the determined analyte, e.g., by comparing it to references in order to provide a diagnostic, prognostic, environmental-relevant, agricultural-relevant or analytically-relevant conclusion depending on the aim of the determination of the analyte.

The term "determining" as used herein encompasses any kind of qualitative or quantitative determinations of the analyte. A qualitative determination aims at determining the presence or the absence of the analyte in the sample whereas quantitative determination aims at determining the amount of the analyte. The quantitative determination, i.e. the determination of the amount, includes determining the absolute amount (e.g. the total amount by weight or number of molecules present in the sample) or a relative amount (e.g., an amount relative to the sample volume (concentration) or a classification such as a score (e.g., "high amount", "low amount" and the like). Typically, determining an analyte comprises determining the presence, absence or amount of said analyte.

The term "analyte" as referred to herein relates to any type of molecule or agent which is suitable for being determined by the method of the invention. It will be understood that such a molecule or agent may have a size and/or structure that allows binding of the first and second binding agents referred to herein. Moreover, there may be upper size limitations as well since the first and second binding agents as well as the linking agent must be capable of exert their functions as described elsewhere herein in detail. Typically, the analyte referred to herein is a biological molecule such as a protein, a peptide, a nucleic acid, e.g., a DNA or RNA or a small molecule such as a lipid or metabolite, a polyketide including, e.g., flavonoids and isoflavonoids, an isoprenoid including, e.g., terpenes, sterols, steroids, carotenoids, xanthophylls, a carbohydrate, a phenylpropanoide, an alcaloide, a benzenoide, an indole, a porphyrine, a hormone, a vitamin, a cofactor, a lignin, a glucosinolate, a purine, a pyrimidine, a nucleoside, a nucleotide, an alcohol, an alkane, an alkene, an alkine, an aromatic compound, a ketone, an aldehyde, a carboxylic acid, a ester, an amine, an imine, an amide, a cyanide, an amino acid, a thiol, a thioester, a phosphate ester, a sulfate ester, a thioether, a sulfoxide or an ether. The small molecule may be, e.g., a toxin. However, the method of the invention can also be used for determining viruses or even bacterial cells as analytes. Analytes to be determined by the present invention may also be molecules which are present in environmental samples and which may be useful as indicators for, e.g., environmental pollution, agriculture or other environmental conditions. Typically, said analyte is a protein, peptide, virus, bacterial cell or small molecule, preferably, small molecule toxin.

The term "sample" as used herein relates to any part or aliquot of a composition comprising or suspected to comprise the analyte to be determined. Such a sample may be a biological sample, typically, isolated from an organism, such as a body fluid or biopsy sample, or may be a composition comprising organism such as a cultured cells. Typically, said biological sample is investigated by the method of the invention for medical purposes, such as diagnosing or predicting a disease or medical condition. Moreover, the sample may be an environmental sample or an artificial sample. An environmental sample may be derived from any non-biological, natural source, e.g., environmentally occurring solutions such as water or from compositions such as soil. An artificial sample may be a sample obtained from an artificial source, e.g., a product composition which may be manufactured or an intermediate composition which may occur during a manufacturing process for a product. Such artificial samples may be investigated, e.g., for quality control purposes or for determining the amount of specific ingredients. Typically, said sample in accordance with the method of the present invention is a biological sample, preferably, a body fluid or biopsy sample.

The term "sensor element" as used in accordance with the present invention comprises a solid support having a surface which is covered by an anchor layer. Moreover, the sensor element shall further comprise a molecular arrangement as described above, i.e. a first binding agent which is capable of specifically binding to the analyte, which is anchored in the anchor layer and a second binding agent which is capable of specifically binding to the analyte when bound to the first binding agent, which is immobilized on the solid support and which comprises at least one magnetic label. Further, a magnetic tunnel junction shall be in functional proximity to the second binding agent such that a signal can be elicited if at least one magnetic label of the second binding agent is in functional proximity to said magnetic tunnel junction.

The term "anchor layer" as referred to herein encompasses any molecular layer that is capable of associating molecules and, in particular, the first binding agent according to the invention. The association of the first binding agent referred to in accordance with the present invention shall allow molecular movements of the first binding agent or any anchoring molecule attached thereto within the anchor layer or on its surface. The said anchor layer is, typically, a lipid layer or a lipid bi-layer. The skilled artisan is well aware of how a solid support can be covered by such an anchor layer and which lipids can be used to create a lipid layer or lipid bi-layer. Typical lipid layers or lipid bi-layers suitable for use as an anchor layer according to the invention may include for example a phospholipid layer or phospholipid bi-layer. Said phospholipid layer or phospholipid bi-layer may particularly comprise one or more phosphatidylcholine(s), such as 1-oleoyl-2-palmitoyl-phosphatidylcholine, 1,2-dioleoyl-sn-glycero-3-phosphocholine and/or 1,2-dioleoyl-sn-glycero-3-[(N-(5-amino-1-carboxypentyl)iminodiacetic acid)succinyl], or a mixture of one or more phosphatidylcholine(s) and cholesterol. Moreover, supported lipid bilayers and tethered bilayer lipid membranes may find use as an anchor layer according to the invention. These are commonly used model lipid membranes known in the art.

The term "solid support" as used herein relates to a solid composition of matter which can serve as a basis for immobilizing molecules and, in particular, the second binding agent. A solid support may comprise inorganic or organic compounds or both. Typically, suitable inorganic compounds for a solid support may be selected from the group consisting of: silica, porous glass, aluminosilicates, borosilicates, metal oxides (e.g. aluminum oxide, iron oxide, nickel oxide) and clay containing one or more of these. Alternatively, the solid support may comprise an organic compound such as a cross-linked polymer. Non-limiting examples of a suitable cross-linked polymer may be selected from the group consisting of: polyamide, polyether, polystyrene and mixtures thereof. The skilled artisan is well aware of how to select a suitable solid support based on the kind of sample to investigated, the method envisaged for detection of the analyte, the kind of magnetic label to be used for detecting the analyte and/or the kind of second binding agent used for the sensor element. The solid support shall also comprise a magnetic tunnel junction in functional proximity to a second binding agent immobilized on the said solid support.

Under "functional proximity" it will be understood that the magnetic tunnel junction is located with respect to the second binding agent in a distance and at a place that allow generation of a signal elicited by the at least one magnetic label of the second binding agent. Said signal may be altered if said first binding agent becomes part of a complex together with the second binding agent and the analyte as defined elsewhere herein.

The sensor shall further comprise a first binding agent which is capable of specifically binding to the analyte. Said first binding agent shall be anchored in the anchor layer.

The term "first binding agent" referred to herein relates to a molecule which is capable of specifically binding to the analyte, i.e. a molecule which does not bind to and, thus, does not cross-react with other molecules than the analyte suspected to be present in the sample. Specific binding, in principle, can be tested by techniques well known in the art including screening assays for identifying agents specifically binding an analyte from libraries comprising different candidate agents.

Molecules which may be used, preferably, as first binding agents being capable of specifically binding a desired analyte may be antibodies. Antibodies as binding agents as meant in accordance with the present invention encompass to all types of antibodies which, preferably, specifically binds to the analyte. Preferably, an antibody of the present invention may be a monoclonal antibody, a polyclonal antibody, a single chain antibody, a chimeric antibody or any fragment of such antibodies being still capable of specifically binding to the analyte. Such fragments comprised by the term antibody as used herein encompass a bispecific antibody, a synthetic antibody, an Fab, F(ab)2 Fv or scFv fragment or a chemically modified derivative of any of these antibody fragments. Antibodies or fragments thereof, in general, which specifically bind to a desired analyte can be obtained by using methods which are described, e.g., in Harlow and Lane "Antibodies, A Laboratory Manual", CSH Press, Cold Spring Harbor, 1988. Monoclonal antibodies can be prepared by the techniques which comprise the fusion of mouse myeloma cells to spleen cells derived from immunized mammals and, preferably, immunized mice (Köhler 1975, Nature 256, 495, and Galfré 1981, Meth. Enzymol. 73,3). The skilled person is well aware of how specific binding can be tested by techniques well known in the art, such as immunological or biochemical techniques including immunoassays, cell sorting or Western blotting or plasmon surface resonance measurements.

Further, molecules which may be used, preferably, as first binding agents being capable of specifically binding a desired analyte may be aptamers. An aptamer as a binding agent in accordance with the present invention may be an oligonucleic acid or peptide molecule that binds to a specific target analyte (Ellington 1990, Nature 346 (6287): 818-22). Bock 1992, Nature 355 (6360): 564-6). Oligonucleic acid aptamers are engineered through repeated rounds of selection or the so-called systematic evolution of ligands by exponential enrichment (SELEX technology). Peptide aptamers usually comprise of a variable peptide loop attached at both ends to a protein scaffold. This double structural constraint shall increase the binding affinity of the peptide aptamer into the nanomolar range. Said variable peptide loop length is, preferably, composed of ten to twenty amino acids, and the scaffold may be any protein having improved solubility and compacity properties, such as thioredoxin-A. Peptide aptamer selection can be made using different systems including, e.g., the yeast two-hybrid system (see e.g., Hoppe-Seyler 2000, J Mol Med. 78 (8): 426-30). Also encompassed according to the present invention are any fragments of said aptamers that are still capable of specifically binding to the analyte. Said fragments can be used in isolated form or may be part of fusion molecules, i.e. molecules comprising said aptamer fragment as well as other parts such as linker moieties or adapter molecules. The skilled person is well aware of how specific binding can be tested by techniques well known in the art, such as plasmon surface resonance measurements.

Molecules which may be used, preferably, as first binding agents being capable of specifically binding a desired analyte may also be receptor molecules. A receptor molecule as a binding agent as referred to in accordance with the present invention is, typically, a protein that specifically binds to a ligand and which become activated upon ligand binding to exert its biological function. Such a receptor molecule or a fragment thereof being still capable of specifically binding to the ligand may also be used as a binding agent in accordance with the present invention for the ligand as analyte or a molecule which is derived from the ligand but still capable of being bound by the receptor molecule or fragment thereof such as antagonistically or agonistically acting variants of a ligand. Preferably, the receptor molecule envisaged as a binding agent in accordance with the present invention may be a transmembrane type receptor protein, such as a G-protein coupled receptor, e.g., a metabolic receptor, an enzyme linked receptor such as a receptor tyrosine kinase, e.g., a growth factor receptor, an immunological receptor, such as a virus receptor, a cell surface antigen, a T cell receptors, e.g., CD4, CD3 or CD8, or a MHC protein, a cell adhesion molecule, such as an integrin, cadherin, selectin or syndecan, a neuronal receptor or a pathogen receptor such as Toll-like receptors. The receptor molecule may also be a nuclear receptor protein such as a nuclear hormone receptor, e.g., the glucocorticoid receptor, retinoic acid receptor or thyroid hormone receptor. The skilled person is well aware of receptor molecules or fragments thereof which specifically bind to an analyte to be determined or fragments thereof. Moreover, specific binding can be tested by techniques well known in the art, such as plasmon surface resonance measurements.

Yet, molecules which may be used, preferably, as first binding agents being capable of specifically binding a desired analyte may also be ligand molecules. A ligand molecule as a binding agent as referred to in accordance with the present invention is, typically, a protein or peptide that specifically binds to a receptor molecule and which activates upon receptor binding the said receptor molecule. Such a ligand molecule or a fragment thereof being still capable of specifically binding to the receptor molecule may also be used as a binding agent in accordance with the present invention for the receptor as analyte or a molecule which is derived from the receptor molecule but still capable of being bound by the ligand molecule or fragment thereof such as soluble variants of a receptor. Moreover, a ligand may also be any antigen which may be used to determine a certain antibody in a sample of an organism. Preferably, the ligand molecule envisaged as a binding agent in accordance with the present invention may be a peptide hormone, a neurotransmitter, a growth factor, such as angiopoietin, a BMP, a neutrotrophic factor, EGF, an epiphrin, EPO, a FGF, a GDNF, a GDF, insulin or an insulin-like growth factor, a TGF, a neutrophin, a VEGF, a cytokine, such as an interleukin, interferon, lymphokine, monokine, colony stimulating factor or chemokine, a extracellular matrix protein such as fibronectin, vitronectin, collagen, ankyrin or laminin, or the like. The skilled person is well aware of ligand molecules or fragments thereof which specifically bind to an analyte to be determined or fragments thereof. Moreover, specific binding can be tested by techniques well known in the art, such as plasmon surface resonance measurements.

Further preferably, the first binding agent may be a Designed Ankyrin Repeat Protein (DARPin). DARPins are genetically engineered antibody mimetic proteins which can be designed to achieve highly specific and high-affinity target protein binding. They are derived from natural ankyrin repeat proteins. Typically, DARPins comprise at least three repeat modules, of which the most N- and the most C-terminal modules (also referred to as "caps") protect the hydrophobic core of the protein (Binz 2003, Journal of Molecular Biology. 332 (2): 489-503).

Typically, said first binding agent is selected from the group consisting of: an antibody or fragment thereof, an aptamer, a receptor molecule or fragment thereof, and a ligand molecule or fragment thereof. More typically, it is an antibody or fragment thereof or an aptamer.

Moreover, the first binding agent in accordance with the present invention shall be anchored in the anchor layer. An anchored first binding agent as referred to in accordance with the present invention shall, typically, be associated with or be within the anchor layer. Accordingly, it is, typically, limited in its movements to essentially in two dimensions in space.

Preferably, said first binding agent is anchored in the anchor layer via an anchoring molecule, more preferably, a lipid. Suitable lipids for anchoring a first binding agent according to the present invention depend on the nature of the anchor layer and can be chosen by the skilled artisan without further ado, The anchoring molecule may be typically linked to at least one, preferably, a plurality of, first binding agent via a linker molecule. Suitable linker molecules allow for attaching many first binding agents and at least one anchoring molecules. Preferably, a suitable linker molecule may be a cyclo-DNA molecule. Thus, a plurality of first binding agents may be anchored to the anchor layer via the same anchoring molecule.

Typically, the amount of said first binding agent exceeds the amount of said second binding agent by a factor between 10 and 100, between 20 and 80, between 30 and 70 or between 40 and 60.

The sensor shall also comprise a second binding agent which is capable of specifically binding to the analyte when bound to the first binding agent. The said second binding agent shall be immobilized on the solid support. Moreover, there shall be functional proximity between the magnetic tunnel junction and the second binding agent as described elsewhere herein. Accordingly, the second binding agent, likewise, shall be in functional proximity to the magnetic tunnel junction. Moreover, the second binding agent shall comprise at least one magnetic label.

The term "second binding agent" which is capable of specifically binding to the analyte relates to a molecule which is capable of specifically binding to the analyte or the analyte when bound to the first binding agent, i.e. a molecule which does not bind to and, thus, does not cross-react with other molecules than the analyte suspected to be present in the sample or the complex of the analyte and the first binding agent. Typically, said second binding agent is selected from the group consisting of: an antibody or fragment thereof, an aptamer, a receptor molecule or fragment thereof, and a ligand molecule or fragment thereof. More typically, it is an antibody or fragment thereof or an aptamer. Typically, the second binding agent is from the same of the aforementioned molecule classes as the first binding agent. The definitions for an antibody or fragment thereof, an aptamer, a receptor molecule or fragment thereof, and a ligand molecule or fragment thereof made in accordance with the first binding agent apply mutatis mutandis for the second binding agent.

The second binding agent shall be immobilized on the solid support. Immobilization of said second binding agent, preferably, is a permanent immobilization. Suitable techniques for immobilizing the second binding agent to the solid support depend on the nature of the solid support and/or the nature of the second binding agent and are well known to the skilled artisan. It will be understood that upon immobilization the second binding agent shall not be capable of moving its position within or outside the anchor layer. However, the second binding agent shall be capable of carrying out bending and other molecular movements or conformational changes required to bind to the analyte or to a first binding agent bound to the analyte.

The interaction of the second binding agent and the analyte and first binding agent shall be, preferably, a reversible interaction. It is envisaged that the association rate is typically larger than the dissociation rate such that complexes of first binding agent, analyte and second binding agent remain stable over a certain time allowing for their determination by detection the presence or abundance or the occurrence rate of the at least one magnetic label within proximity to the position of the second binding agent during said time window.

The second binding agent may comprise the at least one magnetic label as a part of the molecule. Alternatively, the at least one magnetic label may be linked to the second binding molecule by a linker. Permanent linkage as well as reversible linkage are envisaged in accordance with the present invention. Reversible linkage may be achieved, e.g., by using biotin-streptavidin based molecular adapter systems well known in the art.

It will be understood that the at least one magnetic label is, typically, located outside functional proximity if the second binding agent is in an unbound state and becomes located in functional proximity if the second binding agent is part of the complex. Accordingly, conformational changes of the second binding agent and/or the at least one magnetic label shall cause an altered position of the said magnetic label with respect to the magnetic tunnel junction. An alteration of the signal generated by the magnetic tunnel junction, in this case, the detection of a signal or the abundance of a signal, shall be indicative for complex formation and, consequently, for the presence of the analyte in the sample.

Also typically, the said at least one magnetic label is located outside the anchor layer if the second binding agent is in an unbound state and becomes located in the anchor layer if the second binding agent is part of the complex. In this case, the at least one magnetic label is also located outside functional proximity to the magnetic tunnel junction and becomes located in functional proximity within the anchor layer after complex formation. Thus, an alteration of the signal generated by the magnetic tunnel junction, in this case, the detection of a signal or the abundance of a signal, shall be indicative for complex formation and, consequently, for the presence of the analyte in the sample, too.

It will be understood that the at least one magnetic label is, typically, also located in functional proximity to the magnetic tunnel junction with a first occurrence rate if the second binding agent is in an unbound state and becomes in functional proximity to the magnetic tunnel junction with a second occurrence rate if the second binding agent is part of the complex, wherein said second occurrence rate is higher than the first occurrence rate. Without wishing to be bound by theory, the physical and chemical properties of the unbound second binding agent and the second binding agent being part of a complex differ and, thus, molecular movements change and the occurrence rate of the at least one magnetic label in functional proximity to the magnetic tunnel junction is altered as a consequence thereof. Thus, an alteration of the signal generated by the magnetic tunnel junction, in this case, the detection of an altered occurrence rate of the at least one magnetic label in functional proximity to the magnetic tunnel junction shall be indicative for complex formation and, consequently, for the presence of the analyte in the sample.

The term "magnetic label" as used herein relates to molecules which can be detected in functional proximity to a magnetic tunnel junction. Accordingly, a magnetic label is typically envisaged in accordance with the present invention as magnetic label. Typical magnetic labels comprise iron-platinum nanoparticles, iron nanoparticles, nickel nanoparticles or cobalt nanoparticles. The magnetic label may be reversibly or permanently bound to the second binding agent. To this end, the magnetic label may be a molecule which is capable of reversibly binding to the second binding agent or it may be a molecule or moiety which is already part of the said second binding agent. Typically, said magnetic label comprises a linker which can be covalently linked by the linking agent to the solid support.

The term "magnetic tunnel junction" as used herein refers to magneto-responsive detectors such as magnetic tunnel junctions or magnetic spin valves (see, e.g., US 5,981,297; Fernandes 2020, Nanomedicine: Nanotechnology, Biology, and Medicine 30, 102287 or Denmark 2019, Journal of Electronic Materials (48): 4749-4761).

The magnetic tunnel junction as detector of the sensor element, typically, is placed below the solid support and said detector being capable of selectively detecting a signal in functional proximity to said second binding element. Depending on the magnetic label, different detection techniques may be applied. Moreover, depending on the detection technique, it may be necessary to apply a magnetic field to the magnetic tunnel junction.

It will be understood that the detected altered signal from the magnetic label can be subsequently transmitted to an evaluation unit. Said evaluation unit may, preferably, comprise a data processing element, such as a computer, with an implemented algorithm for determining the presence absence or amount of an analyte in the sample based on the detected signal(s) of magnetic label(s).

Such an implemented algorithm may evaluate the measured signals elicited from the magnetic label(s) in a complex of second binding agent, analyte and first binding agent for signal strength, signal duration and other predetermined parameters. Based on said evaluation, truly positive signals can be identified and validated and noise signals can be identified and disregarded for further evaluations. The skilled artisan is well aware of what algorithms may be used and how they can be implemented in the device of the present invention. Preferably, the formation of the complex of first binding agent, analyte and second binding agent is detected by measuring the strength and/or duration of a signal elicited by the at least one magnetic label. More preferably, said signal can be detected for a predetermined characteristic time period. Characteristics of true signals are, preferably, formation within a first time window depending on one or more association constants for the complex comprising the first binding agent, analyte and the second binding agent, persistence for a predefined time window and dissociation within a second time window depending on one or more dissociation constants for the complex comprising the first binding agent, analyte and the second binding agent. Typically, a true signal can be detected for a significantly longer time than a signal elicited by at least one magnetic label comprised by a freely moving first binding agent which randomly passes the anchor layer in proximity to the immobilized second binding agent. The latter one shall only generate a short noise signal.

For a precise evaluation of the signals of the magnetic labels detected in accordance with the method of the present invention and for determining the analyte based thereon, computer-implemented algorithm and, in particular, artificial intelligence algorithms, machine learning algorithms and the like may be applied.

In step (a) according to the method of the preset invention, a sample which comprises the analyte to be determined or which is suspected to comprise the analyte to be determined is brought into contact with a biosensor as specified elsewhere herein in detail.

The term "contacting" as used herein relates to bringing the aforementioned components in physical proximity such that the first and/or second binding agent may bind to the analyte if present in the sample. It will be understood that binding of the first binding agent to the analyte and of the second binding agent to the analyte or the complex of first binding agent and analyte may require time and applying suitable conditions. The skilled person is well aware of what time is required for achieving binding and what conditions need to be applied. For example, the sample and the binding agents may be dissolved or mixed with buffers adjusting salt concentrations and/or pH value. It will be understood that suitable buffers and other auxiliary components which may be applied depend on the binding agents to be used and the chemical nature of the analyte to be determined.

In step (a) of the method of the present invention, the first binding agent and the analyte are also brought into contact to a second binding agent immobilized on the solid support which is capable of specifically binding to the analyte or to the first binding agent when bound to the analyte. Typically, the arrangement on the sensor element comprising first binding agent anchored to the anchoring layer and immobilized second binding agent is provided prior to the application of the sample, However, the sample may be brought into contact with the first binding agent and be applied to an anchor layer comprising the second binding agent immobilized on a solid support as well.

As a result of the aforementioned activities which occur during step (a) of the method of the present invention, an analyte will be bound by the first binding agent. The said complex of analyte and first binding agent shall move within the anchor layer and shall be bound by the second binding agent immobilized on the solid support such that a complex is generated comprising the first binding agent, analyte and second binding agent ("lock-on"). Due to molecular binding kinetics, this complex will persist in proximity to the immobilization position of the second binding molecule on the solid support for a characteristic time window until it dissolves ("lock-off"). The second binding agent applied in the method of the present invention shall comprise at least one magnetic label, typically, either permanently or reversibly bound thereto.

In step (b) of the method of the present invention, the aforementioned complex of first binding agent, analyte and second binding agent is detected based on the signal elicited from the at least one magnetic label which is generated by the magnetic tunnel junction, whereby the analyte is determined.

The term "detecting" as used herein relates to identifying the presence, absence and/or amount of magnetic labels. It will be understood that the magnetic label typically generates a signal that can be measured by the magnetic tunnel junction. The signal strength and/or duration is, typically, indicative for the amount of the magnetic label molecules present in the complex in functional proximity to the magnetic tunnel junction. Preferably, the formation of the complex of first binding agent, analyte and second binding agent is detected by measuring the strength and/or duration of a signal elicited by the at least one magnetic label. More preferably, said signal can be detected for a predetermined characteristic time period. However, detecting as referred to herein may further encompass detecting an altered signal. In particular, it the at least one magnetic label comprised in the second binding agent is in functional proximity to the magnetic tunnel junction with a first occurrence rate and the molecular movement behavior is altered upon complex formation, the complex formation can be detected by a second occurrence rate observable after complex formation. Thus, detection as used herein may also encompass the detection of an altered occurrence rate of the signal(s) elicited by the at least one magnetic label when being in functional proximity to the magnetic tunnel junction.

In the method of the present invention, first and second binding agents may be used for determining different analytes. In such a case, it will be understood that once the complexes for such different analytes are formed, each complex must possess a different magnetic label that is capable of generating a signal on the magnetic tunnel junction that differs in at least one signal characteristic.

In a particular embodiment of the method of the present invention, said solid support is a well or predetermined subdivided detection area. Preferably, each well or predetermined subdivided detection area has immobilized on it a predefined amount of second binding agent. Said method is particularly useful for determining an analyte comprises determining the amount of said analyte. Typically, said amount is determined by counting the wells or predetermined subdivided detection areas in which a complex has been formed. According to this particular embodiment, the presence or absence or the amount of analyte is detected for each well or predetermined subdivided area. Afterwards, the number of positive wells or predetermined subdivided areas shall be determined. A positive well or predetermined subdivided area shall be one which exhibits characteristic signal for a proper complex formation as described elsewhere herein having a predetermined strength or duration, e.g., a measurable signal above a predetermined strength threshold and/or over a predetermined time window. Based on the positive wells or predetermined subdivided areas and the predefined amount of second binding agent immobilized on each of said wells or predetermined subdivided areas, the amount of analyte can be determined, e.g., by calculations. For example, if each well or predetermined subdivided area contains ideally one second binding agent, the presence of one molecule of analyte in a sample shall generate one complex. Thus, if the presence of a complex in a well or on a predetermined subdivided area is determined, this presence reflects the presence of one analyte present in the sample. Thus, using a predefined amount of second binding agents on isolated items such as wells or any other predetermined subdivided areas, allows for quantitative or semi-quantitative determination of analyte molecules present in a sample. This technique is also called "digital" detection.

In the method of the present invention, in particular, for the purpose of digital detection, said sample is contacted with at least 100 sensor elements, at least 300 sensor elements, at least 500 sensor element, at least 1,000 sensor element, at least 5,000 sensor elements, at least 10,000 sensor element, at least 50,000 sensor elements or at least 100,000 sensor elements. More preferably, the amount of said analyte comprises counting the individual measured signals generated by the magnetic tunnel junctions.

Advantageously, it has been found in accordance with the present invention that using a first binding agent limited in its movements to essentially two dimensions by association to an anchor layer such as a lipid layer in a sandwich assay format improves sensitivity and suppresses undesired background noise. In accordance with the present invention, an immune complex comprising a first binding agent, e.g., antibody, an analyte to be determined, a second binding agent, e.g., antibody, is formed with a certain kinetic, persists over a certain time window and is dissolved with a certain kinetic ("lock-on, lock-off" principle). Moreover, depending on the molecular configuration used, a signal of a certain strength can be expected. Thus, in the method of the present invention a kinetic measurement of complex formation and complex dissolution is determined rather than a single formation event. This allows for dynamic real-time measurements and may even make washing steps and other treatments unnecessary. Due to the use of the anchor layer, noise causing events on the solid support shall also be reduced. Depending on the nature of the detector, it is possible to essentially singularize the complex formation events. In accordance with the present invention, a magneto-responsive detector measuring a signal elicited by a magnetic label is used being associated with a single second binding agent initiating the complex formation in the presence of an analyte molecule, it will be understood that complex formation shall represent the said analyte molecule present in the sample. In such a digital format, the number of signals received by the individual detectors may be used to count the analyte molecules present in the sample.

Thanks to the present invention, determination of an analyte in a sample with an improved sensitivity and specificity is possible. Moreover, even single molecule events may be determined.

The present invention relates to a device for determining an analyte suspected to be present in a sample comprising a sensor element comprising:
(i) an anchor layer which is present on a solid support;
(ii) a first binding agent which is capable of specifically binding to the analyte, which is anchored in the anchor layer;
(iii) a second binding agent which is capable of specifically binding to the analyte when bound to the first binding agent and which is immobilized on the solid support, said second binding agent comprising at least one magnetic label; and
(iv) a magnetic tunnel junction in functional proximity to the second binding agent which generates a signal elicited in proximity to the at least one magnetic label of the second binding agent.

The term "device" as used herein relates to a system comprising the aforementioned components operatively linked to each other as to allow the determination of the analyte according to the method of the invention.

The sensor element in accordance with the present invention may be located in a reaction zone comprised by the device. The reaction zone may either allow directly for sample application or it may be connected to a loading zone where the sample is applied. In the latter case, the sample can be actively or passively transported via the connection between the loading zone and the reaction zone to the reaction zone. Moreover, the reaction zone shall be also connected to a detector. Suitable detectors and detection techniques are described elsewhere herein in detail. The connection between reaction zone and detector shall be such that the detector can detect the magnetic labels. Suitable connections depend on the techniques used for measuring the presence or amount of magnetic label(s). The sensor element also comprises a magnetic tunnel junction in functional proximity to the second binding agent which generates a signal elicited in proximity to the at least one magnetic label of the second binding agent.

The present invention, in general, relates to the use of the device of the invention for determining an analyte suspected to be present in a sample in said sample.

The present invention relates, furthermore, to a kit for determining an analyte suspected to be present in a sample comprising the device of the present invention.

The term "kit" as used herein refers to a collection of components required for carrying out the method of the present invention including the device of the present invention. Typically, the components of the kit are provided in separate containers or within a single container. The container also typically comprises instructions for carrying out the method of the present invention. These instructions may be in the form of a manual or may be provided by a computer program code which is capable of carrying out or supports the determination of the analyte referred to in the methods of the present invention when implemented on a computer or a data processing device. The computer program code may be provided on a data storage medium or device such as an optical storage medium (e.g., a Compact Disc) or directly on a computer or data processing device or may be provided in a download format such as a link to an accessible server or cloud. Moreover, the kit may, usually, comprise analyte solutions with standardized amounts for calibration or validation or other reference amounts. The kit according to the present invention may also comprise further components which are necessary for carrying out the method of the invention such as washing solutions, solvents, and/or reagents required for detection of the magnetic label.

### FIGURES

**Figure 1****:** A schematic view of the sensor arrangement. On the left, a free second binding agent is shown in a "lock-off" stage. The second binding agent is immobilized on the solid support and comprises a magnetic label outside the anchor layer as well as the binding domain, e.g., an antibody fragment. On the right, a complex is shown comprising the first binding agent, e.g., an antibody fragment, which is anchored in the anchor layer via a lipid anchor, an analyte, and the second binding agent ("lock-on" stage). Due to binding of the binding of the second binding agent to the analyte, the magnetic label becomes positioned into the anchor layer and, thus, in functional proximity to the magnetic tunnel junction shown below the solid support. As a consequence a signal or altered signal is generated which can be used to detect the presence of the complex and, hence, the presence of an analyte molecule in the sample.

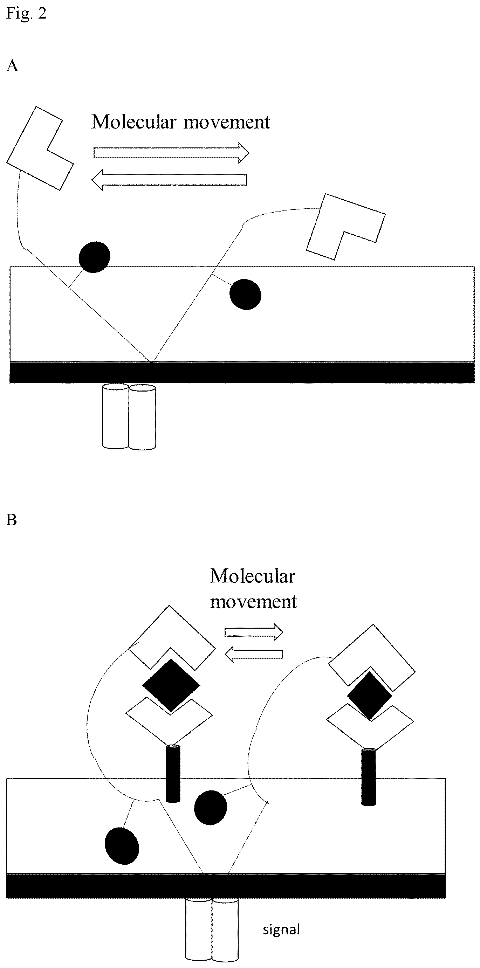

**Figure 2****:** A schematic view of the sensor arrangement. (A) A free second binding agent is shown in a "lock-off" stage. The second binding agent having a binding domain, e.g., an antibody fragment, said second binding agent being immobilized on the solid support and comprises a magnetic label within or outside the anchor layer dependent on the molecular movements which can be made by the second binding agent. In this lock-off stage, the second binding agent shall be capable of making large molecular movements such the occurrence rate of the magnetic label in functional proximity to the magnetic tunnel junction is low. As a consequence, only a limit number of signals occur within a predefined time window. (B) A second binding agent is shown in a complex with an analyte and a first binding agent, i.e. in a "lock-on" stage. In this lock-on stage, the second binding agent shall be capable of making only minor molecular movements such the occurrence rate of the magnetic label in functional proximity to the magnetic tunnel junction is high. As a consequence, only a higher number of signals occur within a predefined time window compared to (A).

## Claims

1. A method for determining an analyte suspected to be present in a sample comprising:
(a) contacting said sample with a sensor element comprising:
(i) an anchor layer which is present on a solid support;
(ii) a first binding agent which is capable of specifically binding to the analyte, which is anchored in the anchor layer;
(iii) a second binding agent which is capable of specifically binding to the analyte when bound to the first binding agent and which is immobilized on the solid support, said second binding agent comprising at least one magnetic label; and
(iv) a magnetic tunnel junction in functional proximity to the second binding agent which generates a signal elicited in proximity to the at least one magnetic label of the second binding agent;
for a time and under conditions which allow for specific binding of the analyte suspected to be present in the sample to the first binding agent and specific binding of the second binding agent to the analyte bound to the first binding agent; and
(b) detecting the formation of the complex of first binding agent, analyte and second binding agent based on an altered signal which is generated by the magnetic tunnel junction whereby the analyte is determined.

2. The method of claim 1, wherein said formation of the complex of first binding agent, analyte and second binding agent is detected by measuring the strength and/or duration of a signal elicited by the at least one magnetic label.

3. The method of claim 2, wherein said signal can be detected for a predetermined characteristic time period.

4. The method of any one of claims 1 to 3, wherein said at least one magnetic label is located outside functional proximity if the second binding agent is in an unbound state and becomes located in functional proximity if the second binding agent is part of the complex.

5. The method of any one of claims 1 to 4, wherein said at least one magnetic label is located outside the anchor layer if the second binding agent is in an unbound state and becomes located in the anchor layer if the second binding agent is part of the complex.

6. The method of claims 1 to 5, wherein said at least one magnetic label is located in functional proximity to the magnetic tunnel junction with a first occurrence rate if the second binding agent is in an unbound state and becomes in functional proximity to the magnetic tunnel junction with a second occurrence rate if the second binding agent is part of the complex, wherein said second occurrence rate is higher than the first occurrence rate.

7. The method of any one claims 1 to 6, wherein said anchor layer is a lipid layer or lipid bi-layer.

8. The method of any one of claims 1 to 7, wherein said first binding agent is selected from the group consisting of: an antibody or fragment thereof, an aptamer, a receptor molecule or fragment thereof, and a ligand molecule or fragment thereof.

9. The method of any one of claims 1 to 8, wherein said second binding agent is selected from the group consisting of: an antibody or fragment thereof, an aptamer, a receptor molecule or fragment thereof, and a ligand molecule or fragment thereof.

10. The method of any one of claims 1 to 9, wherein said determining an analyte comprises determining the amount of said analyte.

11. The method of any one of claims 1 to 10, wherein said sample is contacted with at least 100 sensor elements, at least 300 sensor elements, at least 500 sensor element, at least 1,000 sensor element, at least 5,000 sensor elements, at least 10,000 sensor element, at least 50,000 sensor elements or at least 100,000 sensor elements.

12. The method of claim 11, wherein said determining the amount of said analyte comprises counting the individual measured signals generated by the magnetic tunnel junctions.

13. A device for determining an analyte suspected to be present in a sample comprising a sensor element comprising:
(i) an anchor layer which is present on a solid support;
(ii) a first binding agent which is capable of specifically binding to the analyte, which is anchored in the anchor layer;
(iii) a second binding agent which is capable of specifically binding to the analyte when bound to the first binding agent and which is immobilized on the solid support, said second binding agent comprising at least one magnetic label; and
(iv) a magnetic tunnel junction in functional proximity to the second binding agent which generates a signal elicited in proximity to the at least one magnetic label of the second binding agent.

14. Use of the device of claim 13 for determining an analyte suspected to be present in a sample in said sample.

15. A kit for determining an analyte suspected to be present in a sample comprising the device of claim 13.

## Patentansprüche

1. Verfahren zum Bestimmen eines Analyten, der vermutlich in einer Probe vorliegt, umfassend:
(a) Inkontaktbringen der Probe mit einem Sensorelement, umfassend:
(i) eine Ankerschicht, die auf einem festen Träger vorliegt;
(ii) ein erstes Bindemittel, das spezifisch an den Analyten binden kann, das in der Ankerschicht verankert ist;
(iii) ein zweites Bindemittel, das spezifisch an den Analyten binden kann, wenn es an das erste Bindemittel gebunden ist, und das auf dem festen Träger immobilisiert ist, wobei das zweite Bindemittel mindestens eine magnetische Markierung umfasst; und
(iv) einen magnetischen Tunnelkontakt in funktionaler Nähe zu dem zweiten Bindemittel, der ein Signal erzeugt, das in der Nähe zu der mindestens einen magnetischen Markierung des zweiten Bindemittels ausgelöst wird;
für eine Zeit und unter Bedingungen, die spezifisches Binden des Analyten, der vermutlich in der Probe vorliegt, an das erste Bindemittel und spezifisches Binden des zweiten Bindemittels an den an das erste Bindemittel gebundenen Analyten ermöglichen; und
(b) Nachweisen der Bildung des Komplexes aus erstem Bindemittel, Analyt und zweitem Bindemittel basierend auf einem veränderten Signal, das von dem magnetischen Tunnelkontakt erzeugt wird, wodurch der Analyt bestimmt wird.

2. Verfahren nach Anspruch 1, wobei die Bildung des Komplexes aus erstem Bindemittel, Analyt und zweitem Bindemittel durch Messen der Stärke und/oder Dauer eines Signals, das von der mindestens einen magnetischen Markierung ausgelöst wird, nachgewiesen wird.

3. Verfahren nach Anspruch 2, wobei das Signal für eine vorbestimmte charakteristische Zeitdauer nachgewiesen werden kann.

4. Verfahren nach einem der Ansprüche 1 bis 3, wobei sich die mindestens eine magnetische Markierung außerhalb einer funktionalen Nähe befindet, wenn sich das zweite Bindemittel in einem ungebundenen Zustand befindet, und in funktionale Nähe gelangt, wenn das zweite Bindemittel Teil des Komplexes ist.

5. Verfahren nach einem der Ansprüche 1 bis 4, wobei sich die mindestens eine magnetische Markierung außerhalb der Ankerschicht befindet, wenn sich das zweite Bindemittel in einem ungebundenen Zustand befindet, und in die Ankerschicht gelangt, wenn das zweite Bindemittel Teil des Komplexes ist.

6. Verfahren nach den Ansprüchen 1 bis 5, wobei sich die mindestens eine magnetische Markierung in funktionaler Nähe zu dem magnetischen Tunnelkontakt mit einer ersten Auftretenshäufigkeit befindet, wenn sich das zweite Bindemittel in einem ungebundenen Zustand befindet, und in funktionale Nähe zu dem magnetischen Tunnelkontakt mit einer zweiten Auftretenshäufigkeit gelangt, wenn das zweite Bindemittel Teil des Komplexes ist, wobei die zweite Auftretenshäufigkeit höher ist als die erste Auftretenshäufigkeit.

7. Verfahren nach einem der Ansprüche 1 bis 6, wobei die Ankerschicht eine Lipidschicht oder Lipiddoppelschicht ist.

8. Verfahren nach einem der Ansprüche 1 bis 7, wobei das erste Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

9. Verfahren nach einem der Ansprüche 1 bis 8, wobei das zweite Bindemittel aus der Gruppe ausgewählt ist, die aus Folgendem besteht: einem Antikörper oder Fragment davon, einem Aptamer, einem Rezeptormolekül oder Fragment davon und einem Ligandenmolekül oder Fragment davon.

10. Verfahren nach einem der Ansprüche 1 bis 9, wobei das Bestimmen eines Analyten das Bestimmen der Menge des Analyten umfasst.

11. Verfahren nach einem der Ansprüche 1 bis 10, wobei die Probe mit mindestens 100 Sensorelementen, mindestens 300 Sensorelementen, mindestens 500 Sensorelementen, mindestens 1.000 Sensorelementen, mindestens 5.000 Sensorelementen, mindestens 10.000 Sensorelementen, mindestens 50.000 Sensorelementen oder mindestens 100.000 Sensorelementen in Kontakt gebracht wird.

12. Verfahren nach Anspruch 11, wobei das Bestimmen der Menge des Analyten das Zählen der einzelnen gemessenen Signale, die von den magnetischen Tunnelkontakten erzeugt werden, umfasst.

13. Vorrichtung zum Bestimmen eines Analyten, der vermutlich in einer Probe vorliegt, umfassend ein Sensorelement, umfassend:
(i) eine Ankerschicht, die auf einem festen Träger vorliegt;
(ii) ein erstes Bindemittel, das spezifisch an den Analyten binden kann, das in der Ankerschicht verankert ist;
(iii) ein zweites Bindemittel, das spezifisch an den Analyten binden kann, wenn es an das erste Bindemittel gebunden ist, und das auf dem festen Träger immobilisiert ist, wobei das zweite Bindemittel mindestens eine magnetische Markierung umfasst; und
(iv) einen magnetischen Tunnelkontakt in funktionaler Nähe zu dem zweiten Bindemittel, der ein Signal erzeugt, das in der Nähe zu der mindestens einen magnetischen Markierung des zweiten Bindemittels ausgelöst wird.

14. Verwendung der Vorrichtung nach Anspruch 13 zum Bestimmen eines Analyten, der vermutlich in einer Probe vorliegt, in der Probe.

15. Kit zum Bestimmen eines Analyten, der vermutlich in einer Probe vorliegt, umfassend die Vorrichtung nach Anspruch 13.

## Revendications

1. Procédé de détermination d'un analyte suspecté d'être présent dans un échantillon comprenant :
(a) la mise en contact dudit échantillon avec un élément capteur comprenant :
(i) une couche d'ancrage qui est présente sur un support solide ;
(ii) un premier agent de liaison qui est capable de se lier spécifiquement à l'analyte, qui est ancré dans la couche d'ancrage ;
(iii) un second agent de liaison qui est capable de se lier spécifiquement à l'analyte lorsqu'il est lié au premier agent de liaison et qui est immobilisé sur le support solide, ledit second agent de liaison comprenant au moins un marqueur magnétique ; et
(iv) une jonction à effet tunnel magnétique à proximité fonctionnelle du second agent de liaison qui génère un signal provoqué à proximité de l'au moins un marqueur magnétique du second agent de liaison ;
pendant une durée et dans des conditions qui permettent une liaison spécifique de l'analyte suspecté d'être présent dans l'échantillon au premier agent de liaison et une liaison spécifique du second agent de liaison à l'analyte lié au premier agent de liaison ; et
(b) la détection de la formation du complexe du premier agent de liaison, de l'analyte et du second agent de liaison en se basant sur un signal modifié qui est généré par la jonction à effet tunnel magnétique moyennant quoi l'analyte est déterminé.

2. Procédé selon la revendication 1, dans lequel ladite formation du complexe du premier agent de liaison, de l'analyte et du second agent de liaison est détectée en mesurant l'intensité et/ou la durée d'un signal provoqué par l'au moins un marqueur magnétique.

3. Procédé selon la revendication 2, dans lequel ledit signal peut être détecté pendant une période de temps caractéristique prédéterminée.

4. Procédé selon l'une quelconque des revendications 1 à 3, dans lequel ledit au moins un marqueur magnétique est situé hors d'une proximité fonctionnelle si le second agent de liaison est dans un état non lié et vient se situer à proximité fonctionnelle si le second agent de liaison fait partie du complexe.

5. Procédé selon l'une quelconque des revendications 1 à 4, dans lequel ledit au moins un marqueur magnétique est situé hors de la couche d'ancrage si le second agent de liaison est dans un état non lié et vient se situer dans la couche d'ancrage si le second agent de liaison fait partie du complexe.

6. Procédé selon les revendications 1 à 5, dans lequel ledit au moins un marqueur magnétique est situé à proximité fonctionnelle de la jonction à effet tunnel magnétique avec un premier taux d'occurrence si le second agent de liaison est dans un état non lié et vient se situer à proximité fonctionnelle de la jonction à effet tunnel magnétique avec un second taux d'occurrence si le second agent de liaison fait partie du complexe, dans lequel ledit second taux d'occurrence est supérieur au premier taux d'occurrence.

7. Procédé selon l'une quelconque des revendications 1 à 6, dans lequel ladite couche d'ancrage est une couche de lipides ou une bicouche de lipides.

8. Procédé selon l'une quelconque des revendications 1 à 7, dans lequel ledit premier agent de liaison est choisi dans le groupe constitué par : un anticorps ou un fragment de celui-ci, un aptamère, une molécule réceptrice ou un fragment de celle-ci et une molécule ligand ou un fragment de celle-ci.

9. Procédé selon l'une quelconque des revendications 1 à 8, dans lequel ledit second agent de liaison est choisi dans le groupe constitué par un anticorps ou un fragment de celui-ci, un aptamère, une molécule réceptrice ou un fragment de celle-ci et une molécule ligand ou un fragment de celle-ci.

10. Procédé selon l'une quelconque des revendications 1 à 9, dans lequel ladite détermination d'un analyte comprend la détermination de la quantité dudit analyte.

11. Procédé selon l'une quelconque des revendications 1 à 10, dans lequel ledit échantillon est mis en contact avec au moins 100 éléments capteurs, au moins 300 éléments capteurs, au moins 500 éléments capteurs, au moins 1 000 éléments capteurs, au moins 5 000 éléments capteurs, au moins 10 000 éléments capteurs, au moins 50 000 éléments capteurs ou au moins 100 000 éléments capteurs.

12. Procédé selon la revendication 11, dans lequel ladite détermination de la quantité dudit analyte comprend le comptage des signaux mesurés individuels générés par les jonctions à effet tunnel magnétique.

13. Dispositif de détermination d'un analyte suspecté d'être présent dans un échantillon comprenant un élément capteur comprenant :
(i) une couche d'ancrage qui est présente sur un support solide ;
(ii) un premier agent de liaison qui est capable de se lier spécifiquement à l'analyte, qui est ancré dans la couche d'ancrage ;
(iii) un second agent de liaison qui est capable de se lier spécifiquement à l'analyte lorsqu'il est lié au premier agent de liaison et qui est immobilisé sur le support solide, ledit second agent de liaison comprenant au moins un marqueur magnétique ; et
(iv) une jonction à effet tunnel magnétique à proximité fonctionnelle du second agent de liaison qui génère un signal provoqué à proximité de l'au moins un marqueur magnétique du second agent de liaison.

14. Utilisation du dispositif selon la revendication 13 pour déterminer un analyte suspecté d'être présent dans un échantillon dans ledit échantillon.

15. Kit de détermination d'un analyte suspecté d'être présent dans un échantillon comprenant le dispositif selon la revendication 13.
